# EUROPEAN PATENT SPECIFICATION

(11) **EP 0 688 204 B1**
(45) Date of publication and mention of the grant of the patent: **30.05.2001**
(21) Application number: 93907342.5
(22) Date of filing: 10.03.1993
(51) Int. Cl.: A61K 7/48

(54) **COMPOSITION AND COSMETIC METHOD FOR SKIN**
MITTEL UND VERFAHREN ZUR BLEICHUNG DER HAUT
COMPOSITION ET PROCEDE POUR BLANCHIR LA PEAU

(43) Date of publication of application: 27.12.1995
(73) Proprietor: YALE UNIVERSITY, New Haven CT 06520 (US)
(72) Inventor: PAWELEK, John, Mason, Hamden, CT 06517 (US); BOLOGNIA, Jean, Lynn, North Haven, CT 06473 (US)
(74) Representative: Adams, Nicola
(86) International application number: US9302113
(87) International publication number: WO9420068

(56) References cited:
- EP-A- 0 269 017
- FR-A- 2 152 442
- S.T.N., Serveur de Bases de Données, KARLSRUHE, DE, Fichier Chemical Abstracts, vol 115, n 109447, 1991 see the abstract

## Description

This invention was made with government support under grant number R-814125 awarded by the U. s. Environmental Protection Agency. The government has certain rights in the invention.

### BACKGROUND OF THE INVENTION

The invention relates to compositions and methods for the whitening of skin and hair and for the inhibition of sun-mediated pigmentation and to the use of specified compounds in the manufacture of such compositions.

U. S. Patent 4,990,330 discloses compositions for topical use having melanin synthesis-inhibiting activity comprising kojic acid or its esters and at least one compound selected from the group consisting of azelaic acid, tropolone, oipoic acid, sorbic acid, glucosamine, derivative of glucosamine, tunicamycin, deoxynojirimycin, glutathione, cysteine, hydroquinone, derivative of hydroquinone, dehydroacetic acid, chelidonic acid and lipoamide. Such compositions are disclosed as having excellent human skin-whitening and anti-suntan effects.

### OBJECT OF THE INVENTION

It is an object of the invention to provide a novel, efficient, and safe way of whitening skin and hair in mammals and inhibiting the pigmenting effects of exposure to the sun.

Another object of the invention is to provide novel compositions to effect such whitening and inhibiting of sun-mediated pigmentation.

### SUMMARY OF THE INVENTION

These and other objects are realized in accordance with the present invention pursuant to which there is administered to a mammal, preferably a human, an amount effective to achieve whitening of a compound which depletes glutathione, preferably a selective inhibitor of γ-glutamyl synthetase and more preferably buthionine sulfoximine. The material applied also contains hydroquinone or an alkyl or aralkyl ether of hydroquinone.

Accordingly, the present invention provides a skin-whitening and/or sun-mediated pigmentation-inhibiting composition comprising (a) a compound which depletes glutathione and (b) hydroquinone or an alkyl or aralkyl ether of hydroquinone. The composition may be a skin-whitening and suntan-inhibiting composition. The invention also provides a hair-whitening composition comprising (a) a compound which depletes glutathione and (b) hydroquinone or an alkyl or aralkyl ether of hydroquinone.

The invention further provides a method of cosmetic non-therapeutic whitening of skin which comprises applying to the skin an amount effective therefor of a skin-whitening composition according to the invention. The invention also provides a method of cosmetic non-therapeutic whitening of hair which comprises applying to the hair an amount effective therefor of a hair-whitening composition according to the invention. The invention further provides a method of cosmetic non-therapeutic inhibition of suntanning or other sun-mediated pigmentation which comprises applying to the skin an amount effective therefor of a sun-mediated pigmentation-inhibiting composition according to the invention.

In further aspects, the invention provides the use of (a) a compound which depletes glutathione and of (b) hydroquinone or an alkyl or aralkyl ether of hydroquinone in the manufacture of a composition for whitening skin, the use of (a) a compound which depletes glutathione and of (b) hydroquinone or an alkyl or aralkyl ether of hydroquinone in the manufacture of a composition for inhibiting suntanning or other sun-mediated pigmentation and also the use of (a) a compound which depletes glutathione and of (b) hydroquinone or an alkyl or aralkyl ether of hydroquinone in the manufacture of a composition for whitening hair.

### DETAILED DESCRIPTION OF THE INVENTION

Buthionine sulfoximine is a known compound of the formula

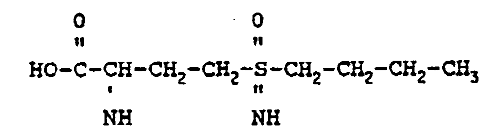

While it has been disclosed that it kills human melanoma cells grown in culture, it has not been disclosed that it has a whitening effect on human skin and hair.

In accordance with the invention, buthionine sulfoximine can be administered in combination with hydroquinone or an alkyl or aralkyl ether thereof, as specified hereinabove; hydroquinone and ethers thereof are known to have a whitening effect. Surprisingly, however, the combination of sulfoximine with hydroquinone or an ether thereof exhibits more than an additive effect.

In such joint administration, the buthionine sulfoximine can be replaced by other compounds which deplete glutathione, particularly those which selectively inhibit γ-glutamyl synthetase.

The following discussion of the compositions of the invention applies also to the compositions used in the methods and manufactured under the uses of the invention.

In the compositions of the present invention (b) is preferably hydroquinone or hydroquinone mono-benzyl ether. As discussed above, (a) is preferably buthionine sulfoximine. The compositions may also comprise an antioxidant, for example, ascorbic acid.

The materials may be applied alone but are preferably administered dissolved or suspended in a carrier which may comprise water, conventional skin moisturizers and creams, and the like. Advantageously, such composition also contains an antioxidant, for which purpose ascorbic acid has been found especially useful.

The active materials can be administered in any manner but are preferably applied topically, e.g., by pouring or rubbing on the skin in the area sought to be whitened.

The buthionine sulfoximine, or other selective inhibitor of γ-glutamyl synthetase, is advantageously applied in a composition comprising a carrier wherein it is present in a concentration of 0.025 to 0.5 mole per liter.

As noted, the compound which depletes glutathione, for example, an γ-glutamyl synthetase inhibitor, (a) is administered in conjunction with (b) hydroquinone or an alkyl or aralkyl ether thereof, e.g. the mono-benzyl ether. The weight ratio of (a):(b) may range from 10:1 to 1:10, advantageously from 5:1 to 1:5 and preferably 1:1.

When an antioxidant (c), such as ascorbic acid, is present desirably it is present at 0.25 to 5% by weight, preferably 1% by weight.

A composition according to the invention may, for example, comprise components (a), (b) and (c) and further contain a carrier, (a) being present at 0.025 to 0.5 mole per liter, the weight ratio of (a): (b) being within the range of from 10:1 to 1:10, and (c) being present at 0.25 to 5% by weight, based on the composition.

The invention will be further described in the following illustrative examples wherein all parts are by weight unless otherwise expressed.

### Example 1

Mouse melanoma cells (4x10⁶) were grown in conventional cultures.

Portions were treated with:
i) nothing;
ii) 2 x 10⁻⁷ M melanocyte stimulating hormone (MSH) plus 10⁻⁴ M isobutylmethylxanthine (IBMX) ;
iii) MSH and IBMX plus 1 x 10⁻⁶ hydroquinone (HQ);
iv) MSH and IBMX plus 5 x 10⁻⁶ M buthionine sulfoximine (BSO):
v) MSH and IBMX plus HQ and BSO.

Following treatment, cells were collected by centrifugation and photographed. Results were as follows: (i) showed no color change and remained white; (ii) darkened considerably as expected, due to the known pigmenting effects of MSH and IBMX; (iii) and (iv) were slightly lighter than (ii); (v) was as white as (i) even though MSH and IBMX were present. The conclusions are that BSO in combination with HQ elicits a strong whitening effect on pigment-producing cells, preventing the induction of pigment by hormones. A further conclusion is that since MSH is known to be a key intermediary in ultraviolet light-induced pigmentation, it is implied that BSO and HQ also elicit inhibition of sun-mediated pigmentation.

### Example 2

Ears and surrounding hair-bearing skin of living C57 black mice were rubbed daily with an aqueous solution containing 25% by weight of glycerol, 0.1 molar TRIS (trihydroxyaminomethane), buffered to p.H 6.8, to which there was added (A) nothing, (B) 4% by weight of hydroquinone, (c) 5% by weight of buthionine sulfoximine, and (D) 4% by weight of hydroquinone plus 5% by weight of buthionine sulfoximine. After 15 days (A) and (C) showed no depigmentation of the surrounding hairs where rubbed, (B) showed noticeable depigmentation and (D) showed marked depigmentation.

### Example 3

The lower backs of living C57 Black mice, from which hair had been removed prior to treatment, were rubbed daily with Acid Mantle Creme (Sandoz Pharmaceuticals Corp.) containing aluminum sulfate, calcium acetate, cetearyl alcohol, glycerin, light mineral oil, methylparaben, purified water, sodium lauryl sulfate, synthetic beeswax, white petrolatum, white potato dextrin, ammonium hydroxide and citric acid, to which there was added (A) nothing (control), (B) 5% by weight buthionine sulfoximine (BSO), (C) 4% by weight hydroquinone (HQ), and (D) 5% by weight of buthionine sulfoximine plus 4% by weight of hydroquinone (BSO/HQ). Treatment was continued until new hair had grown out in the previously plucked regions (about 15 days). Approximately 100 new hairs from the treated areas were removed and scored under a microscope by two independent observers for the percentage of white hairs amongst the black hairs. (A) and (B) showed no white hairs, (C) showed about 2% white hairs, (D) showed about 14% white hairs. Thus BSO in combination with HQ is almost an order of magnitude more effective in eliciting whitening than either agent used alone.

### EXAMPLE 4

Composition and Method for Eliciting Whitening of Human Skin and for Inhibiting the Pigmenting Effects of Ultraviolet Light

Acid Mantle Creme (Sandoz Pharmaceutical Corporation) (or other suitable carriers known to the art) containing 1-4% by weight hydroquinone and 1-5% by weight buthionine sulfoximine.

Directions: Apply daily until the desired whitening or prevention of sun tanning is achieved.

## Claims

1. A skin-whitening and/or sun-mediated pigmentation-inhibiting composition comprising (a) a compound which depletes glutathione and (b) hydroquinone or an alkyl or aralkyl ether of hydroquinone.

2. A hair-whitening composition comprising (a) a compound which depletes glutathione and (b) hydroquinone or an alkyl or aralkyl ether of hydroquinone.

3. A composition according to claim 1 wherein the composition is a skin-whitening and suntan-inhibiting composition.

4. A composition according to any one of claims 1 to 3, wherein (a) is buthionine sulfoximine.

5. A composition according to any one of claims 1 to 4, wherein (b) is hydroquinone or hydroquinone mono-benzyl ether.

6. A composition according to any one of claims 1 to 5, further comprising (c) an antioxidant.

7. A composition according to claim 6, wherein (c) is ascorbic acid.

8. A composition according to claim 6 or claim 7, further containing a carrier, (a) being present at 0.025 to 0,5 mole per liter, the weight ratio of (a): (b) being within the range of from 10:1 to 1:10, and (c) being present at 0.25 to 5% by weight, based on the composition.

9. A method of cosmetic non-therapeutic whitening of skin which comprises applying to the skin an amount effective therefor of a skin-whitening composition according to claim 1 or claim 3 or any one of claims 4 to 8 when dependent, directly or indirectly, on claim 1 or claim 3.

10. A method of cosmetic non-therapeutic whitening of hair which comprises applying to the hair an amount effective therefor of a composition according to claim 2 or any one of claims 4 to 8 when dependent, directly or indirectly, on claim 2.

11. A method of cosmetic non-therapeutic inhibition of suntanning or other sun-mediated pigmentation which comprises applying to the skin an amount effective therefor of a sun-mediated pigmentation-inhibiting composition according to claim 1 or claim 3 or any one of claims 4 to 8 when dependent, directly or indirectly, on claim 1 or claim 3.

12. Use of (a) a compound which depletes glutathione and of (b) hydroquinone or an alkyl or aralkyl ether of hydroquinone in the manufacture of a composition for whitening skin.

13. Use of (a) a compound which depletes glutathione and of (b) hydroquinone or an alkyl or aralkyl ether of hydroquinone in the manufacture of a composition for inhibiting suntanning or other sun-mediated pigmentation.

14. Use of (a) a compound which depletes glutathione and of (b) hydroquinone or an alkyl or aralkyl ether of hydroquinone in the manufacture of a composition for whitening hair.

15. Use according to any one of claims 12 to 14 wherein the composition is a composition according to any one of claims 3 to 8.

## Patentansprüche

1. Eine hautbleichende und/oder die sonnenvermittelte Pigmentierung inhibierende Zusammensetzung, die umfaßt (a) eine Verbindung, die eine Abreicherung von Glutathion bewirkt, und (b) Hydrochinon oder einen Alkyl- oder Aralkylether von Hydrochinon.

2. Eine haarbleichende Zusammensetzung, die umfaßt (a) eine Verbindung, die eine Abreicherung von Glutathion bewirkt, und (b) Hydrochinon oder einen Alkyl- oder Aralkylether von Hydrochinon.

3. Zusammensetzung nach Anspruch 1, wobei die Zusammensetzung eine hautbleichende und eine Sonnenbräunung inhibierende Zusammensetzung ist.

4. Zusammensetzung nach irgendeinem der Ansprüche 1 bis 3, worin (a) Buthioninsulfoximin ist.

5. Zusammensetzung nach irgendeinem der Ansprüche 1 bis 4, worin (b) Hydrochinon oder Hydrochinonmonobenzylether ist.

6. Zusammensetzung nach irgendeinem der Ansprüche 1 bis 5, die außerdem (c) ein Antioxidans umfaßt.

7. Zusammensetzung nach Anspruch 6, worin (c) Ascorbinsäure ist.

8. Zusammensetzung nach Anspruch 6 oder Anspruch 7, die außerdem einen Träger enthält, wobei (a) in 0,025 bis 0,5 Mol pro Liter vorhanden ist, das Gewichtsverhältnis von (a) : (b) im Bereich von 10:1 bis 1:10 liegt und (c) in 0,25 bis 5 Gew.-%, bezogen auf die Zusammensetzung, vorhanden ist.

9. Verfahren zum kosmetischen, nicht-therapeutischen Bleichen von Haut, das das Aufbringen einer dafür wirksamen Menge einer hautbleichenden Zusammensetzung nach Anspruch 1 oder Anspruch 3 oder nach irgendeinem der Ansprüche 4 bis 8, wenn diese direkt oder indirekt von Anspruch 1 oder Anspruch 3 abhängig sind, auf die Haut umfaßt.

10. Verfahren zum kosmetischen nicht-therapeutischen Bleichen von Haar, das das Aufbringen einer dafür wirksamen Menge einer Zusammensetzung nach Anspruch 2 oder nach irgendeinem der Ansprüche 4 bis 8, wenn diese direkt oder indirekt von Anspruch 2 abhängig sind, auf das Haar umfaßt.

11. Verfahren zum kosmetischen nicht-therapeutischen Inhibieren einer Sonnenbräunung oder einer anderen sonnenvermittelten Pigmentierung, das das Aufbringen einer dafür wirksamen Menge einer die sonnenvermittelte Pigmentierung inhibierenden Zusammensetzung nach Anspruch 1 oder Anspruch 3 oder nach irgendeinem der Ansprüche 4 bis 8, wenn diese direkt oder indirekt von Anspruch 1 oder Anspruch 3 abhängig sind, auf die Haut umfaßt.

12. Verwendung von (a) einer Verbindung, die eine Abreicherung von Glutathion bewirkt, und von (b) Hydrochinon oder einem Alkyl- oder Aralkylether von Hydrochinon bei der Herstellung einer Zusammensetzung zum Bleichen der Haut.

13. Verwendung von (a) einer Verbindung, die eine Abreicherung von Glutathion bewirkt, und von (b) Hydrochinon oder einem Alkyl- oder Aralkylether von Hydrochinon bei der Herstellung einer Zusammensetzung zur Inhibierung einer Sonnenbräunung oder einer anderen sonnenvermittelten Pigmentierung.

14. Verwendung von (a) einer Verbindung, die eine Abreicherung von Glutathion bewirkt, und von (b) Hydrochinon oder einem Alkyl- oder Aralkylether von Hydrochinon bei der Herstellung einer Zusammensetzung zum Bleichen von Haar.

15. Verwendung nach irgendeinem der Ansprüche 12 bis 14, wobei die Zusammensetzung eine Zusammensetzung nach irgendeinem der Ansprüche 3 bis 8 ist.

## Revendications

1. Composition blanchissant la peau et/ou inhibant la pigmentation à cause du soleil comprenant (a) un composé qui épuise la glutathione et (b) une hydroquinone ou un alkyl ou aralkyl éther d'hydroquinone.

2. Composition blanchissant les poils comprenant (a) un composé qui épuise la glutathione et (b) de l'hydroquinone ou un alkyl ou aralkyl éther d'hydroquinone.

3. Composition selon la revendication 1 où la composition est une composition blanchissant la peau et inhibant le bronzage.

4. Composition selon l'une quelconque des revendications 1 à 3, où (a) est la buthionine sulfoximine.

5. Composition selon l'une quelconque des revendications 1 à 4, où (b) est hydroquinone ou hydroquinone monobenzyl éther.

6. Composition selon l'une quelconque des revendications 1 à 5, comprenant (c) un antioxydant.

7. Composition selon la revendication 6 où (c) est l'acide ascorbique.

8. Composition selon la revendication 6 ou la revendication 7 contenant de plus un support, (a) qui est présent à 0,025 jusqu'à 0,5 mole par litre, le rapport pondéral de (a) : (b) étant dans la gamme de 10:1 à 1:10 et (c) étant présent à 0,25 à 5% en poids en se basant sur la composition.

9. Méthode de blanchissement cosmétique non thérapeutique de la peau qui comprend l'application, à la peau, d'une quantité efficace pour cela d'une composition blanchissant la peau selon la revendication 1 ou la revendication 3, ou l'une des revendications 4 à 8, dépendant directement ou indirectement de la revendication 1 ou de la revendication 3.

10. Méthode de blanchissement cosmétique non thérapeutique des poils qui comprend l'application, aux poils, d'une quantité efficace dans ce but d'une composition selon la revendication 2 où l'une quelconque des revendications 4 à 8 quand elles sont dépendantes, directement ou indirectement, de la revendication 2.

11. Méthode d'inhibition cosmétique non thérapeutique du bronzage ou autre pigmentation à cause du soleil qui comprend l'application à la peau d'une quantité efficace pour cela d'une composition inhibant la pigmentation due au soleil selon la revendication 1 ou la revendication 3 ou l'une quelconque des revendications 4 à 8 quand elles sont dépendantes, directement ou indirectement, de la revendication 1 ou de la revendication 3.

12. Utilisation (a) d'un composé qui épuise la glutathione et (b) d'hydroquinone ou un alkyl ou aralkyl éther d'hydroquinone dans la fabrication d'une composition pour le blanchissement de la peau.

13. Utilisation (a) d'un composé qui épuise la glutathione et (b) d'hydroquinone ou un alkyl ou aralkyl éther d'hydroquinone dans la fabrication d'une composition pour l'inhibition du bronzage ou autre pigmentation à cause du soleil.

14. Utilisation (a) d'un composé qui épuise la glutathione et (b) d'hydroquinone ou d'un alkyl ou aralkyl éther d'hydroquinone dans la fabrication d'une composition pour le blanchissement des cheveux.

15. Utilisation selon l'une quelconque des revendications 12 à 14, où la composition est une composition selon l'une quelconque des revendications 3 à 8.
